# EUROPEAN PATENT SPECIFICATION

(11) **EP 4 427 012 B1**
(45) Date of publication and mention of the grant of the patent: **15.10.2025**
(21) Application number: 22812767.6
(22) Date of filing: 02.11.2022
(51) Int. Cl.: G01N 1/00, A61L 9/03, A61L 9/12, F24F 8/00

(54) **DEVICE FOR THE DETECTION OF BACTERIA IN THE AIR**
VORRICHTUNG ZUM NACHWEIS VON BAKTERIEN IN DER LUFT
DISPOSITIF DE DÉTECTION DE BACTÉRIES DANS L'AIR

(30) Priority: 03.11.2021 IT 202100027974
(43) Date of publication of application: 11.09.2024
(73) Proprietor: Nutrintech Med Italia S.r.l., 00197 Roma (RM) (IT)
(72) Inventor: BRANDIMARTE, Bruno, 00048 Nettuno RM (IT)
(74) Representative: Tiburzi, Andrea
(86) International application number: PCT/IB2022/060563
(87) International publication number: WO 2023/079460

(56) References cited:
- KR-A- 20120 086 384
- US-A1- 2008 241 875

## Description

### FIELD OF THE INVENTION

The present invention refers to a device for detecting the presence of bacteria or viruses in an ambient air sample. The device specifically takes advantage of the fluorescence of bacteria/viruses to allow their identification.

### BACKGROUND

The problem of being capable to detect the presence of bacteria and viruses, which has always been felt within the most disparate technical fields (food, health, etc.), has become extremely relevant.

In many environments, indeed, a high bacterial or viral load is found, such as in hospital wards and/or in intensive care unit. Said problem is also felt at home, with reference to the presence of patients in quarantine or in pathological conditions which do not require hospitalization.

Therefore, detecting the bacterial and/or viral load found in the ambient air becomes essential both as a data in itself and to determine the need for sanitizing the environment itself.

Indeed, airborne microorganisms, like other chemical agents, may have harmful effects on human health. These are air-transported in the form of bio-aerosols, bound to dust and particles, with the subsequent risk of exposure by inhalation, as well as by contact with contaminated surfaces or objects.

For this reason, it is necessary to assess the environmental contamination by microorganisms. Such assessment is also essential in order to proceed with a correct sanitation of the contaminated air.

The prevention and protection from biological agents are further dealt with at a regulatory level by Legislative Decree 81/08 under title X.

Currently, bacterial detectors configured as laboratory equipment, which perform bacterial analyses on different types of materials, in liquid or solid state, such as water, food or soil samples, are available.

Such apparatuses, however, have important limitations.

First, they do not have the functionality of being capable to detect bacteria in an air sample. Furthermore, they do not allow the overall display of the bacterial flora present, nor even to perform the survey automatically and supply signals which can be used as data for instant reading. KR 2012 0086384 A, US 2008/241875 A1 disclose systems to detect microorganisms in an air sample.

### SUMMARY

The technical problem posed and solved by the present invention is therefore to provide a device for the detection of bacteria and/or viruses in an ambient air sample, allowing to overcome the problems above mentioned with reference to the known art.

This is achieved by a device according to independent claim 1, and by a device according to independent claim 11.

Furthermore, subject of the present invention is a sanitizing system of the air environment comprising an aforementioned bacterial detection device, according to the further independent claim 19.

Secondary characteristics and particular embodiments of the subjects of the present invention are defined in the dependent claims.

The device for the detection of bacteria herein proposed is configured to detect and quantify the presence of gram positive and gram negative viruses and/or bacteria in an ambient air sample.

To achieve such an aim, an automatic device has been studied allowing the detection described and providing an electronic signal capable of directly allowing the overall reading of the bacterial/viral flora present or to control a sanitizer, which is also electronically controlled by a microprocessor.

Other advantages, characteristics and methods of use of the present invention will become apparent from the following detailed description of several embodiments, presented by way of non-limiting examples.

### BRIEF DESCRIPTION OF THE DRAWING

In the following description, reference will be made to the attached Figures, wherein:
- Figure 1 shows a block diagram of a first preferred embodiment of a device for the detection of bacteria and/or viruses in an ambient air sample according to the present invention;
- Figure 2 shows a block diagram of a second preferred embodiment of a device for the detection of bacteria and/or viruses in an ambient air sample according to the present invention; and
- Figure 3 shows a block diagram of a preferred embodiment of an ambient air sanitizing system according to the present invention.

The Figures above indicated are intended solely for illustrative and non-limiting purposes.

### DETAILED DESCRIPTION OF THE INVENTION

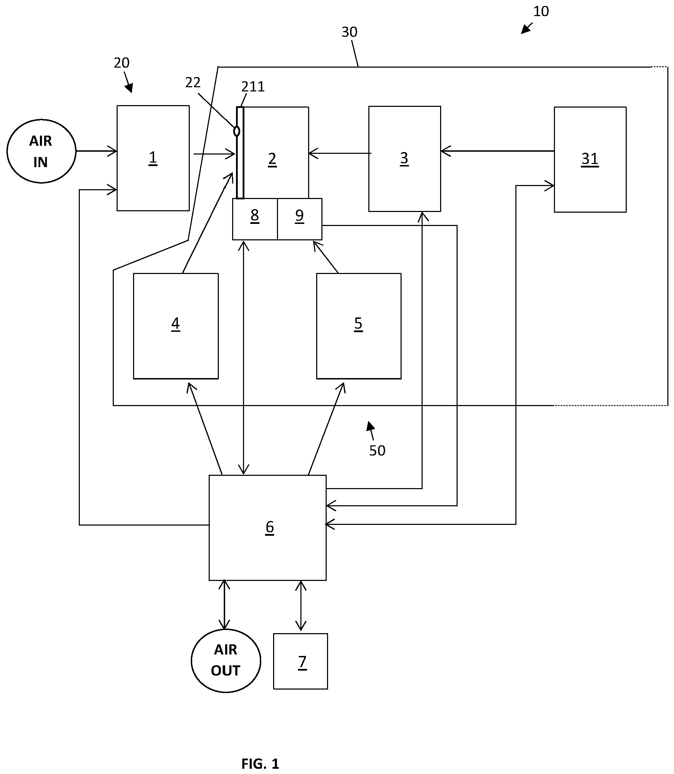

With reference to Figure 1, a preferred embodiment of a device for the detection of bacteria and/or viruses according to the present invention is denoted by the reference number 10.

Device 10 is configured to detect and quantify the presence of bacteria (gram positive and/or gram negative) and/or virus in an ambient air sample.

Referring to the block diagram shown in Figure 1, device 10 substantially comprises a suction unit of the air environment to be analysed connected to a detection chamber.

The suction unit includes a suction air pump 1, configured to perform active sampling of collected air in the environment wherein the device 10 itself is installed. Preferably, the pump 1 has a suction flow rate comprised between 4 and 6 meters³/h (in particular, 5 meters³/h) and a pressure of between 1 and 5 atmospheres (in particular, 2 atmospheres).

The suction unit comprises an inlet region or section IA of the air aspirated by the pump 1, at which filtering means for the micro-powders is preferably installed, to prevent such impurities to enter the device 10.

The action of the suction pump 1 is such that the air collected - and preferably microfiltered - is sent to the detection chamber. The detection chamber has an upper portion, for example a cap with a preferably hemispherical, or pyramidal, or truncated-pyramidal shape, sealing coupled, but reversibly, with a corresponding bottom wall, which can have a circular or polygonal (for example square) plan, depending on the geometry of the cap.

The bottom wall supports at least one filter 2 arranged in such a way as to be hit by the ambient air sample, in such a way that all the sample passes there through. Filter 2 is configured for the interception of viruses/bacteria, which can be replaced after the analysis of the air sample by removing the bottom wall of the detection chamber.

According to preferred embodiments of the device 10 according to the present invention, the bottom wall can be configured to support a plurality of filters (or rather an array of filters) for intercepting viruses/bacteria, preferably four filters. In such case, the bottom wall can have a non-continuous non-unitary conformation and/or a surface for the support of the filters, although it may be divided, or comprise, a respective bottom element for each filter. Such bottom element can be configured as a container tray for the filter, and the bottom elements can be made in such a way as not to be directly connected to each other.

The content below regarding a single filter 2 and the corresponding bottom wall of the specific embodiment herein described, is intended to be applicable, respectively, to any additional available filters and to the corresponding bottom wall (or to the corresponding bottom elements).

The upper portion of the cap carries at least one access opening for the inlet of the air sample aspirated by pump 1, preferably arranged in a central position, for example at the apex of the cap. Filter 2 carries an operating surface 211 facing the air stream entering the detection chamber, which operating surface 211 preferably has the same dimensions as the bottom wall.

According to a preferred variant, the filter 2 is constituted by a cotton wool tablet, for example by a discoidal shape and a circular plan when the detection chamber has a circular plan, with a diameter preferably ranging from 2 to 8 cm (typically equal to 5 cm). With reference to preferred embodiments, the filter 2 rather has a square plan, for example when the bottom wall has a square plan. The bottom wall supporting filter 2 is preferably metallic, and as mentioned has an area corresponding to that of the cotton tablet. The bottom wall is also provided with a plurality of through holes in order to allow the outflow of the air investing filter 2, so as to exit outside the detection chamber after passing through filter 2. Preferably, the diameter of the holes for the passage of air can be comprised between 7 and 9 mm (typically 8 mm).

The operating surface 211 of filter 2 is configured to collect the humid component of the air, which contains bacteria and viruses associated with water particles. In order to obtain or facilitate the condensation of such humid component, cooling means 8 is preferably provided configured to cool the filter 2, in particular to lower the temperature of the operating surface 211.

According to a preferred variant, cooling means 8 comprises a Peltier cell arranged in such a way as to withdraw heat from filter 2.

The cooling of the filter 2 causes (or improves, speeding it up) the condensation of the liquid component contained within the aspirated air passing through the filter 2. In addition, the device 10 can comprise devices for detecting the temperature 22 of the operating surface 211 of filter 2. Preferably, the device 10 further comprises a control unit 6, for example a microprocessor, which controls the temperature of filter 2 (obviously, it is intended to be the temperature of the operating surface 211) using such devices and subsequently adjusts the operation of the cooling means 8, so that the temperature of the filter 2 is kept constant, or in any case within a predetermined operating range that allows the condensation of the humid component of the air. Preferably, the filter 2 is kept at a temperature of + 1 °C, or nonetheless within a range comprised between + 5 °C and -5 °C.

The device 10 further comprises means for dispensing 3 a substance that renders the bacteria and/or viruses coloured, luminous or fluorescent. The means for dispensing 3 is configured to supply such substance at the operating surface 211 of filter 2, in such a way as to interact with the condensed liquid component therein located.

According to the device variants which have a plurality of filters, the configuration is such that preferably at least one of such filters is not sprinkled by the on-site substance for the detection of the bacterial/viral load. In other words, the device can comprise a plurality of means for dispensing 3, each associated with a respective filter except at least one, or delivery means configured to deliver the substance at all filters except at least one.

For example, in a variant which has four filters, one of the latter is not associated or exposed to means for dispensing 3.

The non-sprinkling of at least one filter allows to keep the bacterial/viral load collected thereon unaltered, in order to subsequently analyse it, for example, in a structure remote with respect to the device, following extraction of the such at least one filter from the device. Therefore, such variant of the device allows to subsequently carry out qualitative analyses of the bacteria/viruses collected from the aspirated air sample.

The means for dispensing 3 can comprise, for example, nozzles for sprinkling the operating surface 211 of the filter 2 with the aforementioned substance, which protrude into the internal chamber by means of suitable openings made onto the upper portion of the latter. According to a preferred variant, the means for dispensing 3 comprises a Venturi system (providing the combination of an electronic pump with an air flow sprinkler). Obviously, the means for dispensing 3 is fluidically connected to a tank 31 from which they draw the substance for the detection, such tank 31 can also be provided as integrated in the means for dispensing 3 itself.

The substance used for the detection can vary according to the specific type of bacteria or virus to be detected, and it may be any substance which causes the exhibition of a specific colour, fluorescence, brilliance or other particular luminescence characteristic.

For example, the use of a nucleic acid-based fluorescent dye such as SYTOTM BC, known to make bacteria and viruses fluorescent, can be provided. Molecular fluorochromes (e.g. SYTOTM BC, B - 7277) are high affinity nucleic acid dyes, which easily penetrate both gram positive and gram negative bacteria; the result is a green or orange fluorescent signal, depending on the dye used, exceptionally bright, when lighted by UV light of the right wavelength, which indicates the presence of bacteria.

It is reported that a test carried out with SYTOTM BC nucleic acid on gram positive "Bacillus Cereus" bacteria gave a positive result, since the fluorescence caused in the bacteria was clearly visible using an ultraviolet lamp with a wavelength of only 250 nm.

The device 10 further comprises means for detecting 50 the bacteria/viruses, made fluorescent, trapped in the filter bulk, which includes a photoelectric sensor 5 (for example an Allen-Bradley sensor or a micro-camera also equipped with a magnifying optic selected between 20 and 1000 X), configured to generate an electrical signal corresponding to the overall luminescence detected on the operating surface 211 of filter 2, and send the same to the control unit 6. Furthermore, the means for detecting 50 can comprise devices of lighting 4 preferably ultraviolet, for example UV LED or Wood light lamp, configured to illuminate the operating surface 211 of the filter 2, further highlighting the bacterial flora or viruses present therein and already treated with substance for the detection, improving the operation of the photoelectric sensor 5.

The control unit is configured to process the electrical signal and, depending on its value, generate a corresponding digital signal containing information relating to the quantity detected of bacteria/viruses. The signal can be used for direct reading of information on a screen 7 embedded in the device 10 itself and connected to the control unit 6, or transmitted to external devices using means for transmitting US comprised within the same control unit 6, such as for example a viewer or the microprocessor of a sanitizing device, better described below.

According to a preferred variant, the means for detecting 50 further comprises a video camera 9 to acquire images of the operating surface 211 of the filter 2, connected to the control unit 6. Preferably, the video camera is equipped with optics from 20 X to 1000 X.

In the presence of the video camera, the control unit 6 comprises a screen 7 whereon the acquired images are displayed in real time, preferably together with the reading of the percentage of bacteria/viruses, reproduced by a figure positioned on the side of screen 7.

The delivery means of the substance for the detection and the means for detecting 50 can be arranged within the internal chamber, at appropriate housings or additional openings made at the upper portion of the chamber itself. Furthermore, the control unit 6 of device 10 is configured to control the bacterial/virus detection process depending on the detected quantity of bacteria/virus, by sending the control signals for activation or deactivation to suction pump 1, means for dispensing 3 and means for detecting 50, as well as any cooling means 8.

In order to allow such functionality control of the device on site, the control unit 6 can comprise or be operationally associated with a user interface, for example provided with touch screen. Alternatively, or in addition, signal reception means may be provided in the control unit 6, to allow interaction and control of the device 10 even remotely by using a suitably programmed computer or a specially designed remote control. Preferably, device 10 can be configured to be remotely controlled electronically and/or by a cloud platform.

It is noticeable that all components of the device 10 above described may be contained within a casing or container, for example made of metal or plastic material, which can be provided with support means such as a base, in turn for example metal or plastic.

A further preferred embodiment of the invention detection device is described below. All components listed below may be combined with the embodiments already described, or by replacing their respective components.

The device is mounted in a metal or plastic container, which comprises a base, metal or plastic for its part, to be inserted in the container itself and which supports all components (mechanical, electromechanical and electronic parts), besides the pump. In particular, the base supports the pump, which is connected to the container via a duct, preferably made of silicone. At the inlet section of the duct in the container, or in further upstream sections, an atmospheric particulate filter is positioned. The particulate filter is preferably inserted into an opening container, in order to allow easy replacement. The pump is connected to the inside of the container, again via a duct preferably in silicone, with the inside of a hemispherical cap with a flat and removable bottom, which the filter for the detection of viruses/bacteria is arranged thereon.

The removal of the bottom part of the bell allows the replacement of the (discoid) cotton wool pad containing the bacteria/viruses of the ambient air sample subjected to the measurements.

The upper part of the bell carries access openings for the duct which conveys the flow of air aspirated in by the pump, preferably arranged in a central position, at the apex of the bell, and for the Venturi nozzle of the substance for the detection, which is preferably a fluorescence liquid. In addition, there are housings or further openings at which devices of Ultraviolet LED lighting, the photoelectric sensor and a micro-camera connected to the control microprocessor are arranged.

A touchscreen is placed outside the container and connected to the microprocessor, in order to allow vision of the filter. Preferably, the number corresponding to the quantity of bacteria/viruses detected is also displayed in a corner of the screen. The screen also implements a user interface for on-site control of the device. The base supports a refillable tank for fluorescence fluid and the Venturi dispenser, as well as the pump. Eventually, an electronic board and/or means for sending and receiving data, all connected to the microprocessor, are also positioned on the base to allow the transmission of detected data to external devices, and to allow remote control of the device.

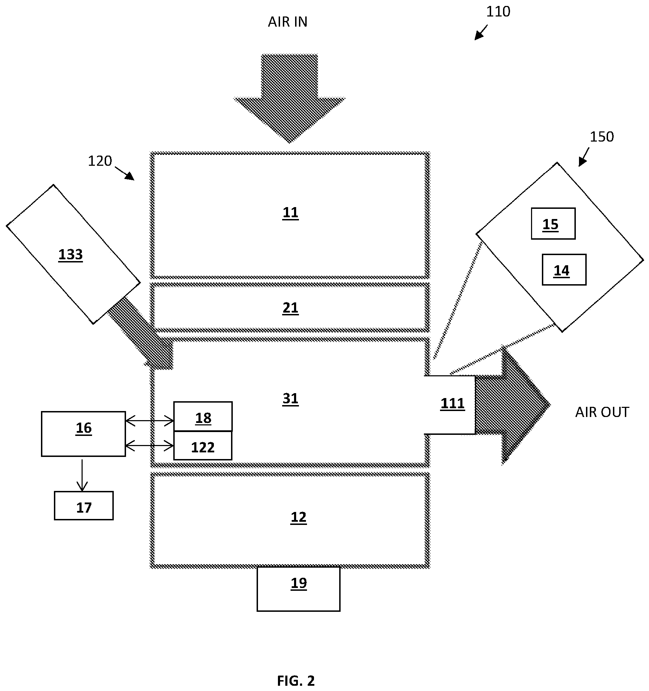

With reference to Figure 2, the present invention provides a still further embodiment 110 of the device for the detection of bacteria and/or viruses contained in the humid component of an ambient air sample, which will be below illustrated exclusively referring to the differences with respect to that already described. In particular, it should be noted that such variant does not have a detection chamber according to that described above, as will be better detailed below.

The device 110 comprises a suction unit 120 of an ambient air sample, which can include a pump 11 according to what has already been described. Downstream of the suction unit 120, a condensation chamber 31 configured to carry out the condensation of the humid component of the aspirated air sample is provided. Preferably, a filter 21 is interposed between the suction unit 120 and the condensation chamber 31 to retain the atmospheric particulate.

The condensation chamber 31 is preferably made of metallic material, in particular steel. Furthermore, chamber 31 is provided with cooling means 18 to lower its temperature and cause condensation of the humid component of the air passing there through.

To this purpose, the device 110 comprises devices for detecting the temperature 122 of the condensation chamber 31 and a control unit 16 connected to the latter and to the cooling means 18. The control unit 16 is programmed to adjust the operation of cooling means 18 in such a way that the temperature of the condensation chamber 31 is kept constant, or within a predetermined operating range, such as to allow the condensation of the humid component of the ambient air sample.

Therefore, according to such variant, the liquid component of the aspirated air sample condenses in the condensation chamber 31, and not on the filter.

After the condensation phase, the air is expelled from the device 110.

Furthermore, the device 110 comprises delivery means 133 of a substance to make the bacteria and/or viruses luminous or fluorescent, configured to supply such substance at the condensation chamber 31, in such a way as to interact with the bacteria and/or viruses contained in the humid component of the ambient air sample already condensed, while the same being inside the aforementioned condensation chamber 31.

A filter 12 is arranged below, preferably to at least partially realize a bottom wall of the condensation chamber 31, in such a way as to collect the liquid component condensed thereon and sprinkled by the substance to cause the luminescence/fluorescence of the bacteria and/or viruses. The filter 12 can consist of a substrate for the collection of viruses/bacteria, such as a membrane or a bacterial filter, preferably with 0,2 µm meshes.

In order to facilitate the collection of the humid component on the filter 12, it is possible to provide pneumatic means 19 (for example a vacuum pump) to create the vacuum in the condensation chamber 31, arranged below the filter 12 itself, in the opposite position with respect to the condensation chamber 31. In other words, the filter 12 is interposed between the condensation chamber 31 and the pneumatic means 19 to create the vacuum, the filter 12 effectively constituting the bottom wall of the condensation chamber 31. Thereby, all the content of the condensation chamber 31 passes through the filter 12 when the means 19 is activated.

In the proposed variant of the device 110, the decoupling is operated between the condensation structure and the substrate for detection of bacteria/viruses, which instead in the variant of device 10 are combined in a cooled filter.

The embodiment of the device 110 is indeed associated with operational steps which firstly provide passage and condensation of the air sample through the condensation chamber 31. Once condensation is completed, the air is expelled from the condensation chamber 31, which contains only the condensed liquid component at this stage. The air is evacuated through a duct or an outlet opening 111, preferably arranged at a lateral wall of the room 31 itself, without passage through the filter 12.

Subsequently, the luminescent/fluorescent dye is added in the condensation chamber 31 combining with the liquid component obtained from condensation and, after an incubation period - having a predetermined duration depending on the volume of the aspirated air sample or the quantity of the humid component - the liquid component thus sprinkled is withdrawn through the filter 12, placed at a bottom wall of the condensation chamber 31. The bacteria trapped on the filter 12 can be observed directly on the surface of the filter 12 itself, for example by means of a microscopy system (e.g. optics 20x or 40x magnification).

To ensure the complete collection of the liquid component present in the condensation chamber 31, pneumatic means 19 can be activated to create the vacuum in the condensation chamber 31, arranged below the filter 12 itself, in such a way that all content of the condensation chamber 31 is filtered there through.

After emptying the condensation chamber 31, the latter must be cleaned or sterilized before using the device 110 again. Also the filter 12 is replaced before the new use, and its removal can be provided to allow access into the condensation chamber 31 to realize the clean thereof.

Further according to such variant, means for detecting 150 configured to identify the luminescence associated with the bacteria/viruses retained above the filter 12 is provided. The means for detecting 150 comprises a photoelectric sensor 15 configured to generate an electrical signal corresponding to the luminescence at the filter 12, which is proportional to the quantity of bacteria and/or viruses contained in the humid component of the ambient air sample. Furthermore, the means for detecting 150 can comprise ultraviolet lighting 14 devices configured to illuminate the filter 12.

Preferably, the device 110 comprises a control unit 16 configured to process the signal generated by the photoelectric sensor 15 and, depending on its value, generate a corresponding digital signal containing information relating to the quantity of bacteria and/or viruses contained in the ambient air sample analysed. Information relating to the quantity of bacteria and/or viruses detected can be displayed on a screen 17.

Preferably, device 110 is configured to be remotely controlled electronically and/or by a cloud platform, for example by establishing a connection with control unit 16.

According to a further aspect of the present invention, a system for the sanitation of ambient air is also provided, preferably for indoor application, which comprises or is operatively associated with the device for the detection of bacteria/viruses as described above.

According to a preferred variant, the device for the detection of bacteria/viruses is mounted on a support or base, for example metal or plastic, to be inserted internally an external case containing the sanitizing device. The base is configured to support all components of the detection device already described, both mechanical, electromechanical and electronic. In particular, the electronic board is positioned on the base, also containing the control microprocessor of the detection device, which will communicate the data to the microprocessor of the sanitizer, which in turn is connected, if necessary, to an external interface or control platform, even remote with respect to the system.

Hereinafter, preferred embodiments of a sanitizing system according to the present invention will be described.

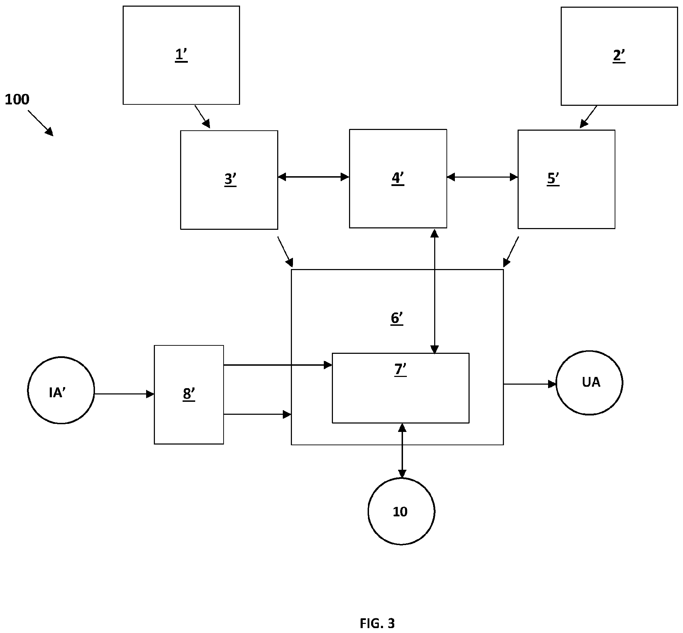

With reference to Figure 3, a preferred embodiment of a system 100 for the sanitation of ambient air from bacteria and/or viruses configured to be operatively connected with, or associated with, a device for the detection of such bacteria and/or viruses as already described, with which data and/or control signals can be exchanged.

Even more preferably, the system 100 can have mechanical connection means with the aforementioned device, or a dedicated housing for containing the same. Such variant results in a sanitizing system physically integrated with the detection device.

Therefore, according to preferred embodiments, the system 100 can equip the device for the detection of such bacteria and/or viruses.

System 100 is configured to deliver predetermined quantities of a bactericidal and/or virucidal substance in the ambient air, which will be hereinafter referred to more generically as a sanitizing substance. The sanitizing substance is a substance in the liquid state that kills viruses and/or bacteria, suitable for being vaporized for diffusion in the ambient air as described below.

Referring to the block diagram shown in Figure 3, the system 100 substantially comprises a containment group of the sanitizing substance, a vaporizer device 6' configured to supply the sanitizing substance in vaporized form by respective delivery means, a suction/emission unit of the ambient air and a control group 4'. All components of the system 100 can be housed within a containment body or casing, which allows an easy transportation and positioning of the system itself. Preferably, said casing is made as a metal or plastic container with a capacity of at least 4 dm³.

According to preferred embodiments, also the device for the detection of bacteria and/or viruses can be comprised within the containment casing. Preferably, in use, the containment group is located at an upper portion of the containment casing, while the vaporizer device 6' is located to a lower portion. Even more preferably, the containment casing has means of access there inside at the upper portion, for example at least one opening closed by a removable plug, in order to access one or more tanks of the containment group for the purpose of cleaning and maintenance (e.g. for a replacement of the same). Similarly, the same means of access can be provided at the lower portion, to allow maintenance and cleaning of the vaporizer 6' (e.g. maintenance and removal operation of the possible tank of the vaporizer device).

Obviously, the containment casing carries at least one opening AI' for the inlet of the air environment to be sanitized and at least one UA outlet opening for the exit of the sanitized ambient air.

The containment group of the sanitizing substance comprises at least one tank, configured to contain a sanitizing substance already suitable for dispensing and in communication with the vaporization group, in particular with a refillable tank, by means of a respective flow regulation valve.

According to a preferred variant of the invention, which involves the use of sanitizing substance wherein there is a sanitizing agent in a concentration not suitable for dispensing, the containment group comprises two tanks: a first tank 1' for containing the sanitizing substance, and a second tank 2' for the containment of a diluent liquid substance, such as water or hydro alcoholic solution, which represent the solvent necessary for the dilution of the sanitizing substance so that the sanitizing agent reaches a predetermined concentration value based on the volume of air to be sanitized, and is suitable for dispensing. The first tank 1' configured for the containment of the sanitizing substance is in fluid communication with a refillable tank of the vaporizer device 6' via a first delivery valve 3', configured to adjust the quantity of sanitizing substance to be transferred to the vaporization group based on the volume of air to be sanitized. The second tank 2' is in fluid communication with the same refillable tank of the vaporization device 6 via a second dispensing valve 5', configured to adjust the quantity of diluent substance to be transferred to the refillable tank to obtain the dilution of the sanitizing substance as provided and planned. In particular, the first and second tanks 1', 2' have a capacity of at least 1000 cm³.

In particular, a sensor for the detection of the concentration of the sanitizing agent can be provided in the first tank 1'.

The vaporizer device 6', as mentioned, is preferably provided with in turn a refillable tank of the sanitizing substance, for example with a capacity of 30 cm³, which is in communication with the containment tank of the sanitizing substance or first tank 1. The vaporizer 6' is configured for the emission of the sanitizing substance in the form of dry vapour by suitable delivery means.

It should be noted that the connections between the tanks and the arrangements herein described are realized by means of silicone tubes with sealed junction points, to ensure their sealing.

According to a preferred variant of the invention, the vaporizer device 6' can be realized in compliance with what is described in the Italian patent no. 102018000006391.

In this case, the vaporizer device comprises: a support body; a containment tank of the sanitizing substance to be vaporized; withdrawal means removably connectable to the tank; delivery means of the substance in fluid connection with the withdrawal means and shaped in order to allow the delivery of the substance in accordance with predetermined direction and speed parameters; first mechanical or electromechanical vaporization means of the vibration type, configured to apply vibrations to the substance contained in the tank in such a way as to bring it into a state of dry vapour; second heating vaporization means, configured to yield thermal energy to the substance contained in the tank, in such a way as to bring it into a dry vapour state; and a control unit configured to control the implementation of the first vaporization means and/or of the second vaporization means in accordance with a predetermined dispensing program. The predetermined dispensing program defines at least frequency and/or duration values of the vibrations applied by the first vaporization means and/or temperature and duration values of the heating operated by said second vaporization means. The predetermined delivery program is associated with the type of substance to be delivered.

The first and second vaporization means can be activated in conjunction or independently according to an automatic or manual selection. The tank, the delivery means, the first vaporization means and/or the second vaporization means are removably connectable to the support body, in such a way as to realize a modular device. In particular, the first vaporization means is configured to apply vibrations at an ultrasonic frequency higher than 5 MHz and to generate an energy density of at least 10 W/cm², while the second vaporization means is configured to bring the temperature of the sanitizing substance to be delivered to at least 250 °C. In particular, a sensor for the detection of the amount of sanitizing substance can be provided in the tank of the vaporizer.

According to a further preferred variant of the invention, the vaporizer device 6' can be realized in compliance with what is described in the European patent application EP3806940A1, which claims the priority of the aforementioned Italian patent.

This vaporizer device comprises all the characteristics listed above, and in particular provides the first vaporization means to be configured to apply vibrations to the unifying substance at an ultrasonic frequency comprised between 2,5 and 5,5 MHz generating an energy density between 10-13 W/cm2, while the second vaporization means comprises magnetic induction media supply by current at a frequency between 15 and 25 KHz and configured to bring the temperature of the substance to at least 250 °C, in such a way as to bring the sanitizing substance contained within the tank in the dry vapour state. The configuration of the vaporizer is such as to realize the vaporization of the liquid sanitizing substance in the form of dry saturated vapour in period less than 1 second.

As mentioned, the sanitation system 100 further comprises a suction/emission unit, comprising means of suction of ambient air and means of emission of the same ambient air, added with the vaporized sanitizing substance.

The suction means is configured to suck a predetermined quantity of ambient air, for example by means of a pump, and convey it into an internal passage chamber through a dedicated duct. The inlet of the ambient air to system 100 can be realized by means of an opening IA in the containment casing, preferably equipped with a filter 8' for the retaining of particulate matter and fine dust. The internal passage chamber faces the delivery means of the vaporizer 6', which deliver the sanitizing substance vaporized on the aspirated flow of ambient air.

The internal passage chamber further communicates with the means of emission, which provides for the discharge of the air sanitized outside through an outlet opening UA.

According to a preferred variant of the invention, the duct conveying the flow of aspirated ambient air to the internal passage chamber is in fluid communication with the virus/bacteria detection sensor. The connection is such that the sensor sucks an air sample, analyses it and then re-emit it at the same duct, upstream of the internal passage chamber. Alternatively, the analysed air sample can be emitted directly into the external environment.

With regard to the control group 4', this can be realized by means of a single system microprocessor controlling the activation of the suction unit/emission, the vaporizer 6' and the delivery valves 3', 5'. The microprocessor 4' can be in data communication also with the bacterial/viral load detection device, to make the system fully automatic and avoid the intervention of the human operator.

In particular, the microprocessor 4' can be programmed to automatically manage the filling of the refillable tank of the vaporizer 6' by the diluent of the second tank 2' when the concentration achieved by the sanitizing active component (measured in micrograms or milligrams per cubic meter) is above a determined threshold value. Furthermore, the filling of the refillable tank of the vaporizer 6 from the first and/or second tank 1', 2' can be controlled when the amount of sanitizing solution contained therein is lower than a predetermined threshold value.

Alternatively, or in addition to what has already been described, the vaporizer 6' can embed an additional own microprocessor 7', which exchanges data and control signals with the system microprocessor 4'. According to possible variants of embodiment of the system 100, interface means can be provided for on-site control of the microprocessor(s) 4', 7' by an operator. Alternatively, or in addition, the microprocessor(s) 4', 7' are provided with wireless communication means, to allow remote control (e.g. activation, deactivation and setting/adjustment of operating parameters) thereof. Furthermore, thanks to the presence of the microprocessor(s) 4', 7' which electrically control all the system components, it is possible to set automatic operating programs according to different application purposes (e.g. sanitation of a hospital environment with high bacterial/viral load, or otherwise sanitation of a low-risk home environment, etc.).

In other words, preferred variants of the sanitizing system of the invention provide that the 7' microprocessor (or the 4' system microprocessor) can be preprogrammed, or controlled according to a program stored therein, which adjusts concentration and timing of the vaporizations. Furthermore, the system microprocessor 4' can automatically control and manage the parameters of the sanitation process according to the environmental measurements carried out by means of the possible sensor for the detection of bacterial/viral load.

In other words, the whole system can be managed by a remote cloud platform both for the supply of sanitized air and for the monitoring the environment contamination.

By way of example, it should be noted that a sanitation program may provide a vaporization time for each dispensing of sanitizing substance ranging between about 10 and 20 seconds (typically equal to 15 seconds), with a period between one dispensing and the next ranging between 60 and 120 minutes (typically 90 minutes). The system microprocessor 4' can control the activation and the operating parameters of the system depending on the bacterial/viral load detected by the detection device.

It should be noted that the sanitizing system according to the present invention provides power supply from the mains by means of a separate power supply, realized in compliance with CE standards for medical purposes.

For the purposes of the correct operation of the sanitizing system according to the invention, it is suggested to position the containment casing at a high altitude with respect to the walking surface of the environment to be sanitized, preferably at a height of 2 meters, and distant from human subjects (also in this case, a distance of at least 2 meters is preferable).

For the purpose of a correct positioning, a shelf or a rack may therefore be provided, placed in a suitable position.

As regards the dispensing of the disinfectant in the environment, by way of example and not exhaustively, a dose of 1 cm³ of disinfectant diluted in 1 or 2 cm³ of water for each cubic meter of environment to be sanitized is provided (this particular example refers the use of sodium hypochlorite-based disinfectant, such as Amuchina ^{®}).

## Claims

1. Device (10) for the detection of bacteria and/or viruses contained in the humid component of an ambient air sample, comprising:
- a suction unit (20) of an ambient air sample; and
- a detection chamber (30) connected to said suction unit (20) and comprising:
▪ at least one filter (2) arranged in such a way that to be hit by the ambient air sample, and carrying an operating surface (211) facing the ambient air sample entering the detection chamber (30);
▪ means for dispensing (3) a substance to make the bacteria and/or viruses luminous or fluorescent, configured to supply said substance at said operating surface (211) in such a way as to interact with the bacteria and/or viruses contained in the humid component condensed thereon; and
▪ means for detecting (50) bacteria and/or viruses comprising a photoelectric sensor (5) configured to generate an electrical signal corresponding to the luminescence of said operating surface (211), which is proportional to the quantity of bacteria and/or viruses contained in the humid component,
further comprising cooling means (8) configured to lower the temperature of said operating surface (211) in order to obtain the condensation of the humid component of the ambient air sample upon the latter.

2. Device (10) according to the preceding claim, comprising devices for detecting the temperature (22) of said operating surface (211) and a control unit (6) connected to the latter and to said cooling means (8), programmed to adjust the operation of said cooling means (8) in such a way that the temperature of said operating surface (211) is kept constant, or within a predetermined operating range, such as to allow the condensation of the humid component of the air.

3. Device (10) according to claim 1 or 2, wherein said detection chamber (30) has an upper portion, carrying an opening for the inlet of the ambient air sample aspirated by said suction unit, which is configured to be reversibly sealing coupled with a bottom wall configured to support said filter (2).

4. Device (10) according to the preceding claim, wherein said upper portion is shaped like a cap with a preferably hemispherical shape, and said bottom wall has a circular plan.

5. Device (10) according to claim 3 or 4, wherein said bottom wall is provided with a plurality of through holes to allow the outflow of the air sample.

6. Device (10) according to one of the preceding claims, wherein said means for detecting (50) comprises ultraviolet lighting devices (4) configured to illuminate said operating surface (211).

7. Device (10) according to one of the preceding claims, comprising a control unit (6) configured to process said electrical signal and, depending on its value, generate a corresponding digital signal containing information relating to the quantity of bacteria and/or viruses contained in the ambient air sample.

8. Device (10) according to the preceding claim, comprising a screen (7) on which information relating to the amount of bacteria and/or viruses detected is displayed.

9. Device (10) according to one of the preceding claims, configured to be remotely controlled electronically and/or from a cloud platform.

10. Device (10) according to one of the preceding claims, comprising a plurality of filters (2) each comprising its own operating surface (211), wherein the configuration is such that the operating surface (211) of at least one of said filters (2) is not sprinkled by said substance.

11. Device (110) for the detection of bacteria and/or viruses contained in the humid component of an ambient air sample, comprising:
▪ a suction unit (120) of an ambient air sample;
▪ a condensation chamber (31) connected to said suction unit (120) and configured to realize the condensation of the humid component of the ambient air sample;
▪ delivery means (133) a substance to make the bacteria and/or viruses luminous or fluorescent, configured to supply said substance at said condensation chamber (31) in such a way as to interact with the bacteria and/or viruses contained in the humid component of the ambient air sample;
▪ at least one filter (12) arranged to realize at least partially a bottom wall of said condensation chamber (31), configured in such a way as to collect the humid component condensed and sprinkled with the substance to make the bacteria and/or viruses luminous or fluorescent; and
▪ means for detecting (150) bacteria and/or viruses comprising a photoelectric sensor (15) configured to generate an electrical signal corresponding to the luminescence at said filter (12), which is proportional to the quantity of the bacteria and/or viruses contained in the humid component of the ambient air sample,
said device (110) further comprising cooling means (18) configured to lower the temperature of said condensation chamber (31) to realize the condensation of the humid component of said ambient air sample.

12. Device (110) according to the preceding claim, comprising devices for detecting the temperature (122) of said condensation chamber (31) and a control unit (16) connected to the latter and to said cooling means (18), programmed to adjust the operation of said cooling means (18) in such a way that the temperature of said condensation chamber (31) is kept constant, or within a predetermined operating range, such as to allow the condensation of the humid component of the ambient air sample.

13. Device (110) according to claim 11 or 12, wherein said condensation chamber (31) is provided with an outlet duct or opening (111) for the draining of the ambient air sample, preferably arranged at a lateral wall of said condensation chamber (31), the configuration being such that the ambient air sample does not pass through said filter (12) for its draining from the device (110).

14. Device (110) according to one of claims 11 to 13, wherein said means for detecting (150) comprises ultraviolet lighting devices (4) configured to illuminate said filter (12).

15. Device (110) according to one of claims 11 to 14, comprising pneumatic means (19) to realize the vacuum in said condensation chamber (31), arranged in such a way that said filter (12) is interposed between said condensation chamber (31) and said pneumatic means (19), in such a way that all the content of said condensation chamber (31) passes through said filter (12) when said pneumatic means (19) is activated.

16. Device (110) according to one of claims 11 to 15, comprising a control unit (16) configured to process said electrical signal and, depending on its value, generate a corresponding digital signal containing information relating to the quantity of bacteria and/or viruses contained in the ambient air sample.

17. Device (110) according to the preceding claim, comprising a screen (17) on which information relating to the quantity of bacteria and/or viruses detected is displayed.

18. Device (110) according to one of claims 11 to 17, configured to be remotely controlled electronically and/or from a cloud platform.

19. Sanitizing system (100) of the ambient air comprising:
- a device (10; 10') according to one of the preceding claims;
- a containment group (1', 2') of a virucidal/bacterial sanitizing substance in the liquid state;
- a vaporizer device (6') configured to receive the sanitizing substance from said containment group (1', 2') and comprising delivery means configured to deliver the sanitizing substance in vaporized form;
- means of suction of ambient air;
- means of emission of the sanitized ambient air; and
- a control group (4') configured to control the activation of said containment group (1', 2'), vaporizer device (6'), means of suction and means of emission,
the overall configuration of the system (100) being such that:
said means of suction is configured to suck in a predetermined quantity of ambient air and convey it into an internal passage chamber facing said delivery means of said vaporizer (6') in such a way that the sanitizing substance is vaporized on the flow of ambient air aspirated,
said internal passage chamber being further communicating with said means of emission so that the aspirated and sanitized ambient air is discharged outside.

## Patentansprüche

1. Vorrichtung (10) zum Nachweis von Bakterien und/oder Viren, die in der feuchten Komponente einer Umgebungsluftprobe enthalten sind, umfassend:
- eine Absaugeinheit (20) für eine Umgebungsluftprobe; und
- eine Detektionskammer (30), die mit der Saugeinheit (20) verbunden ist und Folgendes umfasst:
▪ mindestens einen Filter (2), der so angeordnet ist, dass er von der Umgebungsluftprobe getroffen wird, und eine Arbeitsfläche (211) trägt, die der in die Detektionskammer (30) eintretenden Umgebungsluftprobe zugewandt ist;
▪ Mittel zur Abgabe (3) einer Substanz, um die Bakterien und/oder Viren leuchtend oder fluoreszierend zu machen, die so konfiguriert sind, dass sie die Substanz an der Arbeitsfläche (211) so abgeben, dass sie mit den Bakterien und/oder Viren, die in der darauf kondensierten feuchten Komponente enthalten sind, in Wechselwirkung treten; und
▪ Mittel zum Nachweis (50) von Bakterien und/oder Viren, die einen fotoelektrischen Sensor (5) umfassen, der so konfiguriert ist, dass er ein elektrisches Signal erzeugt, das der Lumineszenz der Arbeitsfläche (211) entspricht, die proportional zur Menge der in der feuchten Komponente enthaltenen Bakterien und/oder Viren ist,
ferner eine Kühleinrichtung (8) umfasst, die so konfiguriert ist, dass sie die Temperatur der Arbeitsfläche (211) senkt, um die Kondensation der feuchten Komponente der Umgebungsluftprobe auf dieser zu erreichen.

2. Vorrichtung (10) nach dem vorhergehenden Anspruch, mit Vorrichtungen zum Erfassen der Temperatur (22) der Arbeitsfläche (211) und einer Steuereinheit (6), die mit der letzteren und mit den Kühlmitteln (8) verbunden ist und so programmiert ist, dass sie den Betrieb der Kühlmittel (8) so einstellt, dass die Temperatur der Arbeitsfläche (211) konstant oder innerhalb eines vorbestimmten Betriebsbereichs gehalten wird, um die Kondensation der feuchten Komponente der Luft zu ermöglichen.

3. Vorrichtung (10) nach Anspruch 1 oder 2, wobei die Erfassungskammer (30) einen oberen Abschnitt aufweist, der eine Öffnung für den Einlass der von der Saugeinheit angesaugten Umgebungsluftprobe trägt, die so konfiguriert ist, dass sie reversibel abdichtend mit einer Bodenwand verbunden ist, die so konfiguriert ist, dass sie den Filter (2) trägt.

4. Vorrichtung (10) nach dem vorhergehenden Anspruch, wobei der obere Teil wie eine Kappe mit einer vorzugsweise halbkugelförmigen Form geformt ist und die Bodenwand einen kreisförmigen Grundriss aufweist.

5. Vorrichtung (10) nach Anspruch 3 oder 4, wobei die Bodenwand mit einer Vielzahl von Durchgangslöchern versehen ist, um das Ausströmen der Luftprobe zu ermöglichen.

6. Vorrichtung (10) nach einem der vorhergehenden Ansprüche, wobei die Mittel zum Erfassen (50) ultraviolette Beleuchtungsvorrichtungen (4) umfassen, die so konfiguriert sind, dass sie die Arbeitsfläche (211) beleuchten.

7. Vorrichtung (10) nach einem der vorhergehenden Ansprüche, umfassend eine Steuereinheit (6), die so konfiguriert ist, dass sie das elektrische Signal verarbeitet und in Abhängigkeit von seinem Wert ein entsprechendes digitales Signal erzeugt, das Informationen bezüglich der Menge an Bakterien und/oder Viren enthält, die in der Umgebungsluftprobe enthalten sind.

8. Vorrichtung (10) nach dem vorhergehenden Anspruch, umfassend einen Bildschirm (7), auf dem Informationen über die Menge der nachgewiesenen Bakterien und/oder Viren angezeigt werden.

9. Vorrichtung (10) nach einem der vorhergehenden Ansprüche, die so konfiguriert ist, dass sie elektronisch und/oder von einer Cloud-Plattform aus ferngesteuert werden kann.

10. Vorrichtung (10) nach einem der vorhergehenden Ansprüche, umfassend eine Vielzahl von Filtern (2), die jeweils ihre eigene Arbeitsfläche (211) aufweisen, wobei die Konfiguration so ist, dass die Arbeitsfläche (211) von mindestens einem der Filter (2) nicht von der Substanz besprüht wird.

11. Vorrichtung (110) zum Nachweis von Bakterien und/oder Viren, die in der feuchten Komponente einer Umgebungsluftprobe enthalten sind, umfassend:
▪ eine Absaugvorrichtung (120) für eine Umgebungsluftprobe;
▪ eine Kondensationskammer (31), die mit der Absaugeinheit (120) verbunden ist und so konfiguriert ist, dass sie die Kondensation der feuchten Komponente der Umgebungsluftprobe realisiert;
▪ Zufuhrmittel (133) für eine Substanz, um die Bakterien und/oder Viren leuchtend oder fluoreszierend zu machen, die so konfiguriert sind, dass sie die Substanz der Kondensationskammer (31) so zuführen, dass sie mit den Bakterien und/oder Viren, die in der feuchten Komponente der Umgebungsluftprobe enthalten sind, interagieren;
▪ mindestens einen Filter (12), der so angeordnet ist, dass er zumindest teilweise eine Bodenwand der Kondensationskammer (31) bildet, und der so konfiguriert ist, dass er die feuchte Komponente auffängt, die kondensiert und mit der Substanz besprüht wird, die die Bakterien und/oder Viren zum Leuchten oder Fluoreszieren bringt; und
▪ Mittel zum Nachweis (150) von Bakterien und/oder Viren, die einen photoelektrischen Sensor (15) umfassen, der so konfiguriert ist, dass er ein elektrisches Signal erzeugt, das der Lumineszenz an dem Filter (12) entspricht und proportional zur Menge der Bakterien und/oder Viren ist, die in der feuchten Komponente der Umgebungsluftprobe enthalten sind, wobei die Vorrichtung (110) ferner eine Kühleinrichtung (18) umfasst, die so konfiguriert ist, dass sie die Temperatur der Kondensationskammer (31) senkt, um die Kondensation der feuchten Komponente der Umgebungsluftprobe zu realisieren.

12. Vorrichtung (110) nach dem vorhergehenden Anspruch, umfassend Vorrichtungen zum Erfassen der Temperatur (122) der Kondensationskammer (31) und eine Steuereinheit (16), die mit der letzteren und mit der Kühleinrichtung (18) verbunden ist und so programmiert ist, dass sie den Betrieb der Kühleinrichtung (18) so einstellt, dass die Temperatur der Kondensationskammer (31) konstant oder innerhalb eines vorbestimmten Betriebsbereichs gehalten wird, so dass die Kondensation der feuchten Komponente der Umgebungsluftprobe möglich ist.

13. Vorrichtung (110) nach Anspruch 11 oder 12, wobei die Kondensationskammer (31) mit einem Auslasskanal oder einer Auslassöffnung (111) für die Ableitung der Umgebungsluftprobe versehen ist, der/die vorzugsweise an einer Seitenwand der Kondensationskammer (31) angeordnet ist, wobei die Konfiguration so ist, dass die Umgebungsluftprobe nicht durch den Filter (12) für ihre Ableitung aus der Vorrichtung (110) läuft.

14. Vorrichtung (110) nach einem der Ansprüche 11 bis 13, wobei die Mittel zum Erfassen (150) Ultraviolett-Beleuchtungsvorrichtungen (4) umfassen, die so konfiguriert sind, dass sie den Filter (12) beleuchten.

15. Vorrichtung (110) nach einem der Ansprüche 11 bis 14, mit einer pneumatischen Einrichtung (19) zum Erzeugen des Vakuums in der Kondensationskammer (31), die so angeordnet ist, dass der Filter (12) zwischen der Kondensationskammer (31) und der pneumatischen Einrichtung (19) angeordnet ist, so dass der gesamte Inhalt der Kondensationskammer (31) durch den Filter (12) hindurchgeht, wenn die pneumatische Einrichtung (19) aktiviert wird.

16. Vorrichtung (110) nach einem der Ansprüche 11 bis 15, umfassend eine Steuereinheit (16), die so konfiguriert ist, dass sie das elektrische Signal verarbeitet und in Abhängigkeit von seinem Wert ein entsprechendes digitales Signal erzeugt, das Informationen bezüglich der Menge an Bakterien und/oder Viren enthält, die in der Umgebungsluftprobe enthalten sind.

17. Vorrichtung (110) nach dem vorhergehenden Anspruch, umfassend einen Bildschirm (17), auf dem Informationen über die Menge der nachgewiesenen Bakterien und/oder Viren angezeigt werden.

18. Vorrichtung (110) nach einem der Ansprüche 11 bis 17, die so konfiguriert ist, dass sie elektronisch und/oder von einer Cloud-Plattform aus ferngesteuert werden kann.

19. Desinfektionssystem (100) für die Umgebungsluft, bestehend aus:
- eine Vorrichtung (10; 10') gemäß einem der vorhergehenden Ansprüche;
- eine Einschlussgruppe (1', 2') eines viruziden/bakteriellen Desinfektionsmittels im flüssigen Zustand;
- eine Verdampfervorrichtung (6'), die so konfiguriert ist, dass sie die desinfizierende Substanz aus der Auffanggruppe (1', 2') aufnimmt, und die eine Abgabeeinrichtung umfasst, die so konfiguriert ist, dass sie die desinfizierende Substanz in verdampfter Form abgibt;
- Absaugung der Umgebungsluft;
- Emissionsmittel für die hygienisierte Umgebungsluft; und
- eine Steuergruppe (4'), die so konfiguriert ist, dass sie die Aktivierung der Einschließungsgruppe (1', 2'), der Verdampfervorrichtung (6'), der Ansaugmittel und der Emissionsmittel steuert,
wobei die Gesamtkonfiguration des Systems (100) derart ist, dass:
das Ansaugmittel so konfiguriert ist, dass es eine vorbestimmte Menge an Umgebungsluft ansaugt und sie in eine innere Durchgangskammer leitet, die dem Abgabemittel des Verdampfers (6') zugewandt ist, so dass die Desinfektionssubstanz mit dem angesaugten Umgebungsluftstrom verdampft wird,
wobei die innere Durchgangskammer weiterhin mit den Emissionsmitteln in Verbindung steht, so dass die angesaugte und desinfizierte Umgebungsluft nach außen abgeleitet wird.

## Revendications

1. Dispositif (10) de détection de bactéries et/ou de virus contenus dans la composante humide d'un échantillon d'air ambiant, comprenant:
- une unité d'aspiration (20) d'un échantillon d'air ambiant; et
- une chambre de détection (30) reliée à ladite unité d'aspiration (20) et comprenant:
▪ au moins un filtre (2) disposé de manière à être touché par l'échantillon d'air ambiant, et comportant une surface de fonctionnement (211) orientée vers l'échantillon d'air ambiant entrant dans la chambre de détection (30);
▪ des moyens de distribution (3) d'une substance destinée à rendre les bactéries et/ou les virus lumineux ou fluorescents, configurés pour fournir ladite substance sur ladite surface de travail (211) de manière à interagir avec les bactéries et/ou les virus contenus dans le composant humide condensé sur celle-ci; et
▪ des moyens de détection (50) de bactéries et/ou de virus comprenant un capteur photoélectrique (5) configuré pour générer un signal électrique correspondant à la luminescence de ladite surface de travail (211), qui est proportionnel à la quantité de bactéries et/ou de virus contenus dans le composant humide,
comprenant en outre des moyens de refroidissement (8) configurés pour abaisser la température de ladite surface de travail (211) afin d'obtenir la condensation de la composante humide de l'échantillon d'air ambiant sur cette dernière.

2. Dispositif (10) selon la revendication précédente, comprenant des dispositifs de détection de la température (22) de ladite surface de travail (211) et une unité de commande (6) reliée à cette dernière et auxdits moyens de refroidissement (8), programmée pour régler le fonctionnement desdits moyens de refroidissement (8) de manière à ce que la température de ladite surface de travail (211) soit maintenue constante, ou dans une plage de fonctionnement prédéterminée, de façon à permettre la condensation de la composante humide de l'air.

3. Dispositif (10) selon la revendication 1 ou 2, dans lequel ladite chambre de détection (30) présente une partie supérieure, portant une ouverture pour l'entrée de l'échantillon d'air ambiant aspiré par ladite unité d'aspiration, qui est configurée pour être couplée de manière réversible et étanche avec une paroi de fond configurée pour supporter ledit filtre (2).

4. Dispositif (10) selon la revendication précédente, dans lequel ladite partie supérieure a la forme d'un capuchon de préférence hémisphérique, et ladite paroi inférieure a un plan circulaire.

5. Dispositif (10) selon la revendication 3 ou 4, dans lequel ladite paroi inférieure est pourvue d'une pluralité de trous traversants pour permettre l'écoulement de l'échantillon d'air.

6. Dispositif (10) selon l'une des revendications précédentes, dans lequel lesdits moyens de détection (50) comprennent des dispositifs d'éclairage ultraviolet (4) configurés pour éclairer ladite surface de travail (211).

7. Dispositif (10) selon l'une des revendications précédentes, comprenant une unité de contrôle (6) configurée pour traiter ledit signal électrique et, en fonction de sa valeur, générer un signal numérique correspondant contenant des informations relatives à la quantité de bactéries et/ou de virus contenus dans l'échantillon d'air ambiant.

8. Dispositif (10) selon la revendication précédente, comprenant un écran (7) sur lequel sont affichées des informations relatives à la quantité de bactéries et/ou de virus détectés.

9. Dispositif (10) selon l'une des revendications précédentes, configuré pour être contrôlé à distance électroniquement et/ou à partir d'une plateforme en nuage.

10. Dispositif (10) selon l'une des revendications précédentes, comprenant une pluralité de filtres (2) comportant chacun sa propre surface de travail (211), dans lequel la configuration est telle que la surface de travail (211) d'au moins un desdits filtres (2) n'est pas arrosée par ladite substance.

11. Dispositif (110) pour la détection de bactéries et/ou de virus contenus dans la composante humide d'un échantillon d'air ambiant, comprenant:
▪ une unité d'aspiration (120) d'un échantillon d'air ambiant;
▪ une chambre de condensation (31) reliée à ladite unité d'aspiration (120) et configurée pour réaliser la condensation de la composante humide de l'échantillon d'air ambiant;
▪ des moyens de distribution (133) d'une substance destinée à rendre les bactéries et/ou les virus lumineux ou fluorescents, configurés pour fournir ladite substance à ladite chambre de condensation (31) de manière à interagir avec les bactéries et/ou les virus contenus dans la composante humide de l'échantillon d'air ambiant;
▪ au moins un filtre (12) agencé pour réaliser au moins partiellement une paroi de fond de ladite chambre de condensation (31), configuré de manière à recueillir le composant humide condensé et saupoudré de la substance pour rendre les bactéries et/ou virus lumineux ou fluorescents; et
▪ des moyens de détection (150) de bactéries et/ou de virus comprenant un capteur photoélectrique (15) configuré pour générer un signal électrique correspondant à la luminescence au niveau dudit filtre (12), qui est proportionnel à la quantité de bactéries et/ou de virus contenus dans la composante humide de l'échantillon d'air ambiant,
ledit dispositif (110) comprend en outre des moyens de refroidissement (18) configurés pour abaisser la température de ladite chambre de condensation (31) afin de réaliser la condensation de la composante humide dudit échantillon d'air ambiant.

12. Dispositif (110) selon la revendication précédente, comprenant des dispositifs de détection de la température (122) de ladite chambre de condensation (31) et une unité de commande (16) reliée à cette dernière et auxdits moyens de refroidissement (18), programmée pour régler le fonctionnement desdits moyens de refroidissement (18) de manière à ce que la température de ladite chambre de condensation (31) soit maintenue constante, ou dans une plage de fonctionnement prédéterminée, de façon à permettre la condensation de la composante humide de l'échantillon d'air ambiant.

13. Dispositif (110) selon la revendication 11 ou 12, dans lequel ladite chambre de condensation (31) est pourvue d'un conduit ou d'une ouverture de sortie (111) pour l'évacuation de l'échantillon d'air ambiant, de préférence disposé sur une paroi latérale de ladite chambre de condensation (31), la configuration étant telle que l'échantillon d'air ambiant ne passe pas par ledit filtre (12) pour son évacuation du dispositif (110).

14. Dispositif (110) selon l'une des revendications 11 à 13, dans lequel lesdits moyens de détection (150) comprennent des dispositifs d'éclairage ultraviolet (4) configurés pour éclairer ledit filtre (12).

15. Dispositif (110) selon l'une des revendications 11 à 14, comprenant un moyen pneumatique (19) pour réaliser le vide dans ladite chambre de condensation (31), disposé de telle sorte que ledit filtre (12) est interposé entre ladite chambre de condensation (31) et ledit moyen pneumatique (19), de telle sorte que tout le contenu de ladite chambre de condensation (31) passe à travers ledit filtre (12) lorsque ledit moyen pneumatique (19) est activé.

16. Dispositif (110) selon l'une des revendications 11 à 15, comprenant une unité de contrôle (16) configurée pour traiter ledit signal électrique et, en fonction de sa valeur, générer un signal numérique correspondant contenant une information relative à la quantité de bactéries et/ou de virus contenus dans l'échantillon d'air ambiant.

17. Dispositif (110) selon la revendication précédente, comprenant un écran (17) sur lequel sont affichées les informations relatives à la quantité de bactéries et/ou de virus détectés.

18. Dispositif (110) selon l'une des revendications 11 à 17, configuré pour être contrôlé à distance électroniquement et/ou à partir d'une plateforme en nuage.

19. Système d'assainissement (100) de l'air ambiant comprenant:
- un dispositif (10; 10') selon l'une des revendications précédentes;
- un groupe de confinement (1', 2') d'une substance désinfectante virucide/bactérienne à l'état liquide;
- un dispositif de vaporisation (6') configuré pour recevoir la substance assainissante dudit groupe de confinement (1', 2') et comprenant des moyens de distribution configurés pour délivrer la substance assainissante sous forme de vapeur;
- un moyen d'aspiration de l'air ambiant;
- les moyens d'émission de l'air ambiant assaini; et
- un groupe de contrôle (4') configuré pour contrôler l'activation dudit groupe de confinement (1', 2'), du dispositif de vaporisation (6'), des moyens d'aspiration et des moyens d'émission,
la configuration globale du système (100) étant telle que:
ledit moyen d'aspiration est configuré pour aspirer une quantité prédéterminée d'air ambiant et l'acheminer dans une chambre de passage interne faisant face au moyen de distribution dudit vaporisateur (6') de manière à ce que la substance assainissant soit vaporisée sur le flux d'air ambiant aspiré,
ladite chambre de passage interne communique en outre avec les moyens d'émission, de sorte que l'air ambiant aspiré et assaini est évacué à l'extérieur.
